(19) Europäisches Patentamt
European Patent Office
Office européen des brevets

(11) **EP 1 150 707 B1**

(12) **FASCICULE DE BREVET EUROPEEN**

(45) Date de publication et mention de la délivrance du brevet:
**27.04.2005 Bulletin 2005/17**

(51) Int Cl.⁷: **A61K 39/00**, A61K 39/385, A61K 48/00, A61P 35/00 // C07K14:26

(21) Numéro de dépôt: **00906412.2**

(22) Date de dépôt: **17.02.2000**

(86) Numéro de dépôt international:
**PCT/FR2000/000394**

(87) Numéro de publication internationale:
**WO 2000/048629 (24.08.2000 Gazette 2000/34)**

(54) **UTILISATION D'UNE PROTEINE OmpA D'ENTEROBACTERIE ASSOCIEE AU PEPTIDE ELAGIGILTV POUR LE TRAITEMENT DE MELANOMES**

VERWENDUNG EINES MIT DEM PEPTID "ELAGIGILTV" ASSOZIIERTEN ENTEROBAKTERIEN-PROTEINS OMPA ZUR BEHANDLUNG VON MELANOMEN

USE OF AN OmpA ENTEROBACTERIUM PROTEIN ASSOCIATED WITH THE ELAGIGILTV PEPTIDE FOR TREATING MELANOMAS

(84) Etats contractants désignés:
**AT BE CH CY DE DK ES FI FR GB GR IE IT LI LU MC NL PT SE**

(30) Priorité: **17.02.1999 FR 9901917**

(43) Date de publication de la demande:
**07.11.2001 Bulletin 2001/45**

(73) Titulaire: **PIERRE FABRE MEDICAMENT**
**92100 Boulogne-Billancourt (FR)**

(72) Inventeurs:
- **RENNO, Toufic**
  **F-74580 Viry (FR)**
- **ROMERO, Pedro**
  **CH-1066 Epalinges (CH)**
- **MICONNET, Isabelle**
  **CH-1005 Lausanne (CH)**
- **CAROTTINI, Jean-Charles**
  **CH-1025 Saint-Suplice (CH)**
- **BONNEFOY, Jean-Yves**
  **F-74350 Le Sappey (FR)**

(74) Mandataire: **Texier, Christian**
  **Cabinet Régimbeau**
  **20, rue de Chazelles**
  **75847 Paris cedex 17 (FR)**

(56) Documents cités:
- **VALMORI D ET AL: "Enhanced generation of specific tumor-reactive CTL in vitro by selected Melan-A/MART-1 immunodominant peptide analogues." JOURNAL OF IMMUNOLOGY, vol. 160, no. 4, 1998, pages 1750-8, XP002139350**
- **HAEUW JF ET AL: "The recombinant Klebsiella pneumoniae outer membrane protein OmpA has carrier properties for conjugated antigenic peptides." EUROPEAN JOURNAL OF BIOCHEMISTRY, vol. 255, 1998, pages 446-454, XP002114947**
- **RAULY I ET AL: "P40: A promising new carrier protein." RESEARCH IN IMMUNOLOGY, vol. 149, no. 1, janvier 1998 (1998-01), page 99 XP002116543**
- **VALMORI D ET AL: "Induction of potent antitumor CTL responses by recombinant vaccinia encoding a Melan-A peptide analogue." JOURNAL OF IMMUNOLOGY, vol. 164, no. 2, 2000, pages 1125-31, XP002139351**
- **KIM S K ET AL: "Induction of HLA class I-restricted CD8+ CTLs specific for the major outer membrane protein of Chlamydia trachomatis in human genital tract infections." JOURNAL OF IMMUNOLOGY, vol. 162, no. 11, 1 juin 1999 (1999-06-01), pages 6855-66, XP002120614**

**Description**

L'invention concerne l'utilisation d'une protéine de membrane OmpA d'entérobactérie, notamment de *Klebsiella pneumoniae,* associée à un antigène ou un haptène pour la préparation d'une composition pharmaceutique destinée à générer ou accroître une réponse T cytotoxique dirigée contre une cellule tumorale. L'invention comprend l'utilisation de ces composés pour la prévention et le traitement des mélanomes, ainsi que des compositions pharmaceutiques comprenant certains de ces composés.

La vaccination est un moyen efficace de prévenir ou de réduire les infections virales ou bactériennes. Le succès des campagnes de vaccination dans ces domaines a permis d'étendre le concept de vaccin jusqu'alors utilisé dans le domaine de l'infectiologie aux domaines du cancer et des maladies auto-immunes. Les antigènes vaccinaux administrés seuls chez l'hôte ne sont souvent pas assez immunogéniques pour induire une réponse immunitaire, et doivent donc être associés à un adjuvant ou couplés à une protéine porteuse pour induire (ou augmenter) leur immunogénicité. Dans ces conditions, seule une réponse immune de type humorale peut être induite. Or, dans le cadre d'une thérapie antivirale, la génération de lymphocytes T cytotoxiques (CTL) capables de reconnaître et de détruire le virus est de toute importance (Bachmann et al., 1994, Eur. J. Immunol., 24, 2228-2236 ; Borrow P., 1997, J. Virol. Hepat., 4, 16-24), comme l'attestent de nombreuses études montrant, *in vivo,* le rôle protecteur des réponses dirigées contre les épitopes viraux (Arvin AM, 1992, J. Inf. Dis., 166, S35-S41 ; Koszinowski et al., 1987 Immunol. Lett., 16, 185-192).

L'importance des réponses CTL a aussi été fortement documentée dans les réponses antitumorales notamment celles dirigées contre les cellules de mélanome (revue dans Rivoltini et al., 1998, Crit. Rev. Immunol. 18. 55-63). Le ou les épitopes CTL (séquences peptidiques interagissant avec les molécules de classe I et présentés aux lymphocytes T CD8+) ont été définis pour plusieurs antigènes.

Cependant, la difficulté réside dans la génération de CTL *in vivo,* due à la faible immunogénicité de ces peptides (Melief, 1992, Adv. Cancer Res., 58, 143-175 ; Nandaz et Sercaz, 1995, Cell, 82, 13-17).

Des recherches s'orientent par conséquent vers l'identification de nouveaux adjuvants, ou de système de délivrance d'antigènes («delivery systein»), permettant d'induire des CTL. Grâce à leur efficacité à présenter les antigènes et à stimuler le système immunitaire, les cellules dendritiques, par exemple, ont été utilisées pour générer des réponses CTL antivirales (Ludewig B et al., 1998, J. Virol., 72, 3812-3818 ; Brossard P. et al., 1997, J. Immunol., 158, 3270-3276) ou anticancéreuses (Nestle F.O. et al., 1998, Nat. Med., 4, 328-332). Les approches ont consisté à charger les cellules dendritiques *ex vivo* avec l'antigène d'intérêt (peptides ou lysat cellulaire) et réimplanter ces cellules chez le patient. D'autres approches consistent à transfecter *ex vivo* les cellules dendritiques avec le gène codant pour l'antigène d'intérêt et à réinjecter ces cellules transfectées (Gilboa E. et al., 1998, Cancer Immunol. Immunother., 46, 82-87). Ces approches ont été utilisées avec succès chez la souris et récemment chez l'homme (Hsu F.J. et al., 1996, Nat. Med., 2, 52-58) mais restent néanmoins complexes dans la mesure où ces cellules doivent être traitées *ex vivo* (transformation des cellules ou internalisation des antigènes) et transplantées dans l'organisme hôte. De même, l'utilisation de particules de type viral (Layton G.T. et al., 1993, J. Immunol., 151, 1097-1107) ou de l'adjuvant incomplet de Freund (IFA) (Valmori et al., Eur. J. Immunol., 1994, 24, 1458-1462) permet de générer des réponses CTL. Toutefois, une vaccination antivirale ou antitumorale réalisée avec des peptides correspondant à des épitopes CTL et en présence d'un tel adjuvant peuvent conduire à un état de tolérance spécifique qui peut conduire dans certains cas à l'effet contraire recherché, c'est-à-dire à une diminution de la réponse immune (Toes et al., Proc. Nat. Acad. Sci. USA, 1996, 93, 7855-7860).

On peut également citer le document Rauly et al. (Research in Immunology, 149, 1, page 99, 1998) qui décrit l'utilisation de la protéine OmpA de Klebsiella pneumoniae, couplé à un épitope dérivé du virus respiratoire syncytial, comme protéine porteuse pour l'obtentionde réponse anticorps de type mixte Th1/Th2 dirigé contre cet épitope, dans le cadre de vaccin humain.

Ainsi, il existe aujourd'hui un grand besoin de disposer d'un composé qui, associé à une molécule, notamment un antigène ou haptène, soit capable de générer des CTL dirigés contre ladite molécule. Un tel composé pourrait en particulier être utilisé pour la préparation d'une composition vaccinale destinée à induire une protection immunitaire de type CTL antiviral, antibactérien, antifongique ou antiparasitaire, ou antitumoral.

De manière surprenante, il a été mis en évidence qu'une protéine de la membrane externe de bactérie à gram négatif, notamment une protéine OmpA d'entérobactérie telle que la protéine P40 de *Klebsiella pneumoniae* (protéine décrite dans WO 95/27787 et WO 96/14415), a la propriété d'éliciter une réponse CTL contre une molécule qui lui est associée de manière covalente ou non, de préférence sans recours à l'addition d'un autre adjuvant.

Ainsi, la présente invention est relative à l'utilisation d'une protéine OmpA d'entérobactérie associé au peptide de séquence SEQ ID N°3 pour la préparation d'une composition pharmaceutique destinée au traitement ou à la prévention des mélanomes malins.

Dans la présente invention, on entendra désigner par le terme « protéine » également les peptides ou les polypeptides et par le terme « OmpA » (pour « Outer Membrane Protéin »), les protéines de la membrane externe de type A.

Par fragment d'une protéine OmpA, on entend désigner en particulier tout fragment de séquence d'acides aminés compris dans la séquence d'acides aminés de la protéine OmpA qui, lorsqu'il est associé à un antigène ou haptène spécifique d'un agent infectieux ou d'une cellule tumorale, est capable de générer ou accroître une réponse T cytotoxique dirigée contre ledit agent infectieux ou ladite cellule tumorale, ledit fragment de la protéine OmpA comprenant au moins 5 acides aminés, de préférence au moins 10 acides aminés ou de manière plus préférée au moins 15 acides aminés.

Par « antigène ou haptène spécifique d'un agent infectieux ou d'une cellule tumorale », on entend désigner en particulier tout composé exprimé par un agent infectieux, tel qu'un virus, une bactérie, une levure, un champignon ou un parasite, par une cellule tumorale, ou un de leurs analogues structuraux, qui seul ou en association avec un adjuvant de l'immunité est capable d'induire une réponse immunitaire spécifique dudit agent infectieux ou de ladite cellule tumorale.

Par analogue d'un antigène ou haptène, on entend désigner en particulier dans la présente description un composé présentant une analogie structurale avec ledit antigène ou haptène capable d'induire une réponse immunologique dirigée contre ledit antigène ou haptène dans un organisme préalablement immunisé avec ledit composé analogue.

Dans un mode de réalisation particulier, l'invention comprend l'utilisation d'une protéine OmpA d'entérobactérie selon l'invention, caractérisée en ce que ladite protéine OmpA d'entérobactérie est obtenue par un procédé d'extraction à partir d'une culture de ladite entérobactérie.

Les procédés d'extraction de protéines de membrane bactériennes sont connus de l'homme de l'art et ne seront pas développés dans la présente description. On peut citer par exemple, mais sans s'y limiter le procédé d'extraction décrit par Haeuw J.H. et al. (Eur. J. Biochem, 255, 446-454, 1998).

Dans un autre mode de réalisation préféré, l'invention comprend également l'utilisation d'une protéine OmpA d'entérobactérie selon l'invention, caractérisée en ce que ladite protéine OmpA d'entérobactérie est obtenue par voie recombinante.

Les méthodes de préparation de protéines recombinantes sont aujourd'hui bien connues de l'homme de l'art et ne seront pas développées dans la présente description, on pourra néanmoins se référer à la méthode décrite dans les exemples. Parmi les cellules utilisables pour la production de ces protéines recombinantes, il faut citer bien entendu les cellules bactériennes (Olins P.O. et Lee S.C., 1993, Recent advances in heterologous gène expression in E. coli. Curr. Op. Biotechnology 4:520-525), mais également les cellules de levure (Buckholz R.G., 1993, Yeast Systems for the Expression of Heterologous Gene Products. Curr. Op. Biotechnology 4:538-542), de même que les cellules animales, en particulier les cultures de cellules de mammifère (Edwards C.P. et Aruffo A., 1993, Current applications of COS cell based transient expression systems. Curr. Op. Biotechnology 4:558-563) mais également les cellules d'insectes dans lesquelles on peut utiliser des procédés mettant en oeuvre par exemple des baculovirus (Luckow V.A., 1993, Baculovirus systems for the expression of human gene products. Curr. Op. Biotechnology 4:564-572).

De manière tout à fait préférée, l'utilisation selon l'invention est caractérisée en ce que ladite entérobactérie est *Klebsiella pneumoniae.*

En particulier, l'invention est relative à l'utilisation selon l'invention, caractérisée en ce que la séquence d'acides aminés de ladite protéine OmpA de *Klebsiella pneumoniae* comprend :

a) la séquence d'acides aminés de séquence SEQ ID N° 2 ; ou
b) la séquence d'acides aminés d'une séquence présentant une homologie d'au moins 80 %, de préférence 90 % et 95 %, après alignement optimal avec la séquence SEQ ID N ° 2.

Par séquence d'acide nucléique ou d'acides aminés présentant une homologie d'au moins 80 % après alignement optimal avec une séquence d'acide nucléique ou d'acides aminés déterminée, on entend désigner une séquence qui après alignement optimal avec ladite séquence déterminée comprend un pourcentage d'identité d'au moins 80 % avec ladite séquence déterminée.

Par «pourcentage d'identité» entre deux séquences d'acide nucléique ou d'acides aminés au sens de la présente invention, on entend désigner un pourcentage de nucléotides ou de résidus d'acides aminés identiques entre les deux séquences à comparer, obtenu après le meilleur alignement, ce pourcentage étant purement statistique et les différences entre les deux séquences étant réparties au hasard et sur toute leur longueur. Les comparaisons de séquences entre deux séquences d'acide nucléique ou d'acides aminés sont traditionnellement réalisées en comparant ces séquences après les avoir alignées de manière optimale, ladite comparaison étant réalisée par segment ou par «fenêtre de comparaison» pour identifier et comparer les régions locales de similarité de séquence. L'alignement optimal des séquences pour la comparaison peut être réalisé, outre manuellement, au moyen de l'algorithme d'homologie locale de Smith et Waterman (1981) [Ad. App. Math. 2:482], au moyen de l'algorithme d'homologie locale de Neddleman et Wunsch (1970) [J. Mol. Biol. 48:443], au moyen de la méthode de recherche de similarité de Pearson et Lipman (1988) [Proc. Natl. Acad. Sci. USA 85:2444], au moyen de logiciels informatiques utilisant ces algorithmes (GAP, BESTFIT, FASTA et TFASTA dans le Wisconsin Genetics Software Package, Genetics Computer Group, 575 Science Dr., Madison, WI, ou encore par les logiciels de comparaison BLAST N ou BLAST P).

Le pourcentage d'identité entre deux séquences d'acide nucléique ou d'acides aminés est déterminé en comparant ces deux séquences alignées de manière optimale par fenêtre de comparaison dans laquelle la région de la séquence d'acide nucléique ou d'acides aminés à comparer peut comprendre des additions ou des délétions par rapport à la séquence de référence pour un alignement optimal entre ces deux séquences. Le pourcentage d'identité est calculé en déterminant le nombre de positions identiques pour lesquelles le nucléotide ou le résidu d'acide aminé est identique entre les deux séquences, en divisant ce nombre de positions identiques par le nombre total de positions dans la fenêtre de comparaison et en multipliant le résultat obtenu par 100 pour obtenir le pourcentage d'identité entre ces deux séquences.

Par exemple, on pourra utiliser le programme BLAST, «BLAST 2 sequences», disponible sur le site http:// www.ncbi.nlm.nih.gov/gorf/b12.html, les paramètres utilisés étant ceux donnés par défaut (en particulier pour les paramètres «open gap penaltie» : 5, et «extension gap penaltie» : 2 ; la matrice choisie étant par exemple la matrice «BLOSUM 62» proposée par le programme), le pourcentage d'identité entre les deux séquences à comparer étant calculé directement par le programme.

Parmi lesdites séquences présentant une homologie d'au moins 80 % avec la séquence OmpA de référence, on préfère les séquences de, ou codant pour des, peptides capables d'induire une activité CTL dirigée spécifiquement contre l'antigène ou haptène qui lui est associée, telle que l'activité CTL mesurée au moyen des techniques standards décrites dans les exemples ci-après.

La présente invention a aussi pour objet l'utilisation selon l'invention, caractérisée en ce que ledit peptide de séquence SEQ ID N°3 est couplé ou mélangé avec ladite protéine OmpA.

L'invention comprend également l'utilisation selon l'invention, caractérisée en ce que ledit peptide de séquence SEQ ID N°3 est couplé par liaison covalente, notamment par couplage chimique, avec ladite protéine OmpA.

Dans un mode de réalisation particulier, l'utilisation selon l'invention est caractérisée en ce qu'il est introduit un ou plusieurs éléments de liaison dans ladite protéine OmpA et/ou dans ledit peptide de séquence SEQ ID N°3 pour faciliter le couplage chimique, de préférence, ledit élément de liaison introduit est un acide aminé.

Selon l'invention, il est possible d'introduire un ou plusieurs éléments de liaison, notamment des acides aminés pour faciliter les réactions de couplage entre la protéine OmpA et ledit peptide de séquence SEQ ID N°3. Le couplage covalent entre la protéine OmpA et ledit peptide de séquence SEQ ID N°3 selon l'invention peuvent être réalisés à l'extrémité N- ou C- terminale de la protéine OmpA. Les réactifs bifonctionnels permettant ce couplage seront déterminés en fonction de l'extrémité de la protéine OmpA choisie pour effectuer le couplage et de la nature dudit antigène ou haptène à coupler.

Dans un autre mode de réalisation particulier, l'utilisation selon l'invention est caractérisée en ce que le couplage entre ledit peptide de séquence SEQ ID N°3 et ladite protéine OmpA est réalisé par recombinaison génétique, lorsque ledit antigène ou haptène est de nature peptidique.

Les conjugués issus d'un couplage à ladite protéine OmpA peuvent être préparés par recombinaison génétique. La protéine chimérique ou hybride (conjugué) peut être produite par des techniques d'ADN recombinant par insertion ou addition à la séquence d'ADN codant pour ladite protéine OmpA d'une séquence codant pour ledit peptide de séquence SEQ ID N°3 de nature protéique.

Les procédés de synthèse des molécules hybrides englobent les méthodes utilisées en génie génétique pour construire des polynucléotides hybrides codant pour les séquences polypeptidiques recherchées. On pourra, par exemple, se référer avantageusement à la technique d'obtention de gènes codant pour des protéines de fusion décrite par D.V. Goeddel (Gène expression technology, Methods in Enzymology, vol. 185, 3-187, 1990).

Sous un autre aspect, l'invention concerne l'utilisation selon l'invention, caractérisée en ce que la composition pharmaceutique comprend une séquence nucléique codant pour ladite protéine hybride, ou comprend un vecteur contenant une séquence nucléique codant pour ladite protéine hybride ou une cellule hôte transformée contenant ladite construction nucléique capable d'exprimer ladite protéine hybride.

L'invention décrit également l'utilisation selon l'invention, pour la préparation d'une composition pharmaceutique destinée à inhiber la croissance de tumeurs.

L'invention décrit la préparation d'une composition pharmaceutique destinée à prévenir ou à traiter les cancers, de préférence les cancers associés à un antigène tumoral.

Parmi les cancers dont les tumeurs expriment un antigène tumoral associé, on peut citer en particulier, mais sans s'y limiter :

- le cancer du sein, du poumon, du colon, et le carcinome gastrique (Kawashima et al., 1999, Cancer Res. 59:431-5) ;
- le mésothéliome, l'ostéosarcome, les cancers du cerveau (Xie et al., 1999, J. Natl. Cancer. Inst. 91:169-75) ;
- le mélanome (Zheuten et al., 1998, Bratilsl. Lek. Listy 99:426-34) ;

- l'adénome cystique du pancréas (Hammel et al., 1998, Eur. J. gastroenterol. Hepatol. 10:345-8) ;
- le cancer colorectal (Ogura et al., 1998, Anticancer Res. 18:3669-75) ;
- le carcinome des cellules rénales (Jantzer et al., 1998, Cancer Res. 58:3078-86) ; et
- le cancer de l'ovaire et du cervix (Sonoda et al., 1996, Cancer. 77:1501-9).

L'invention a en particulier pour objet l'utilisation d'une protéine OmpA d'entérobactérie selon l'invention, pour la préparation d'une composition pharmaceutique vaccinale destinée à prévenir ou à traiter les mélanomes.

L'invention comprend également l'utilisation selon l'invention, caractérisée en ce que ladite composition pharmaceutique est véhiculée sous une forme permettant d'améliorer sa stabilité et/ou son immunogénicité, notamment sous la forme d'un liposome.

De préférence, l'invention comprend l'utilisation selon l'invention, caractérisée en ce que ledit véhicule est un vecteur viral contenant une séquence nucléique codant pour ladite protéine OmpA ledit peptide de séquence SEQ ID N°3, ou ladite protéine hybride, ou une cellule hôte transformée capable d'exprimer ladite protéine OmpA, ledit peptide de séquence SEQ ID N°3, ou ladite protéine hybride.

L'invention comprend en outre l'utilisation selon l'invention, caractérisée en ce que ladite séquence nucléique, ou la séquence nucléique contenue dans ledit vecteur ou ladite cellule hôte transformée, comprend une séquence nucléique choisie parmi la séquence SEQ ID N° 1, ou une séquence présentant une homologie d'au moins 80 % après alignement optimal avec l'une desdites séquences.

La présente invention a donc pour objet l'utilisation d'une protéine OmpA d'entérobactérie associé au peptide de séquence SEQ ID N° 3 : ELAGIGILTV pour la préparation d'une composition pharmaceutique destinée au traitement ou à la prévention des mélanomes malins.

Sous un autre aspect, l'invention a pour objet :

- une composition pharmaceutique caractérisée en ce qu'elle comprend une protéine OmpA d'entérobactérie associée par mélange ou par couplage covalent au peptide de séquence ELAGIGILTV ; ou
- une composition pharmaceutique caractérisée en ce qu'elle comprend une séquence nucléique contenant un acide nucléique codant pour une protéine OmpA d'entérobactérie et un acide nucléique codant pour le peptide de séquence ELAGIGILTV.

Comme défini précédemment pour l'utilisation selon l'invention, ladite composition pharmaceutique selon l'invention pourra comprendre par exemple une protéine OmpA d'entérobactérie couplée par liaison covalente au peptide de séquence ELAGIGILTV par synthèse chimique, à partir d'OmpA recombinante ou obtenue par un procédé d'extraction, ou couplée par recombinaison génétique.

Comme défini également précédemment pour l'utilisation selon l'invention, ladite composition pharmaceutique selon l'invention pourra comprendre par exemple un vecteur comprenant une séquence nucléique contenant un acide nucléique codant pour une protéine OmpA d'entérobactérie et/ou un acide nucléique codant pour le peptide de séquence ELAGIGILTV, ou encore une cellule transformée capable d'exprimer une protéine OmpA d'entérobactérie et/ou le peptide de séquence ELAGIGILTV.

L'invention a également pour objet une composition pharmaceutique caractérisée en ce qu'elle comprend la protéine OmpA de *Klebsiella pneumoniae* de séquence SEQ ID N° 2 ou une protéine dont la séquence présente une homologie d'au moins 80 % après alignement optimal avec la séquence SEQ ID N° 2 associée par mélange ou par couplage au peptide de séquence ELAGIGILTV.

La présente invention a en outre pour objet une composition pharmaceutique caractérisée en ce qu'elle comprend une séquence nucléique contenant un acide nucléique codant pour la protéine OmpA de *Klebsiella pneumoniae* de séquence SEQ ID N° 2 ou une protéine dont la séquence présente une homologie d'au moins 80 % après alignement optimal avec la séquence SEQ ID N° 2 et un acide nucléique codant pour le peptide de séquence ELAGIGILTV.

Selon la présente invention, lesdites compositions seront véhiculées sous une forme permettant d'améliorer sa stabilité et/ou son immunogénicité, telle que sous la forme d'un liposome, ou d'un vecteur viral ou d'une cellule hôte transformée capable d'exprimer ladite protéine OmpA et ledit peptide de séquence ELAGIGILTV.

Selon la présente invention, lesdites compositions seront de préférence contenues dans un milieu pharmaceutiquement acceptable.

Au sens de la présente invention, le milieu pharmaceutiquement acceptable est le milieu dans lequel les composés de l'invention sont administrés, préférentiellement un milieu injectable chez l'homme. Il peut être constitué d'eau, d'une solution aqueuse saline ou d'une solution aqueuse à base de dextrose et/ou de glycérol.

Selon la présente invention, lesdites compositions pourront contenir en outre un détergent.

Les compositions selon l'invention peuvent contenir en outre un détergent, et notamment tout type de tensioactif pharmaceutiquement acceptable, comme par exemple des tensioactifs anioniques, cationiques, non-ioniques ou amphotères. On utilise préférentiellement les détergents Zwittergent 3-12 et l'octylglucopyrannoside et encore plus préférentiellement le Zwittergent 3-14.

L'invention comprend également les compositions selon l'invention, caractérisées en ce qu'elles ne contiennent aucun autre adjuvant permettant d'induire une réponse CTL.

Il est décrit également que, ladite composition pharmaceutique ne contient pas d'adjuvant de l'immunité, en dehors de la protéine OmpA d'entérobactérie ou d'un acide nucléique codant pour la protéine OmpA d'entérobactérie, caractéristique des compositions pharmaceutiques de l'invention.

Les légendes des figures et exemples qui suivent sont destinés à illustrer l'invention sans aucunement en limiter la portée. Légendes des figures :

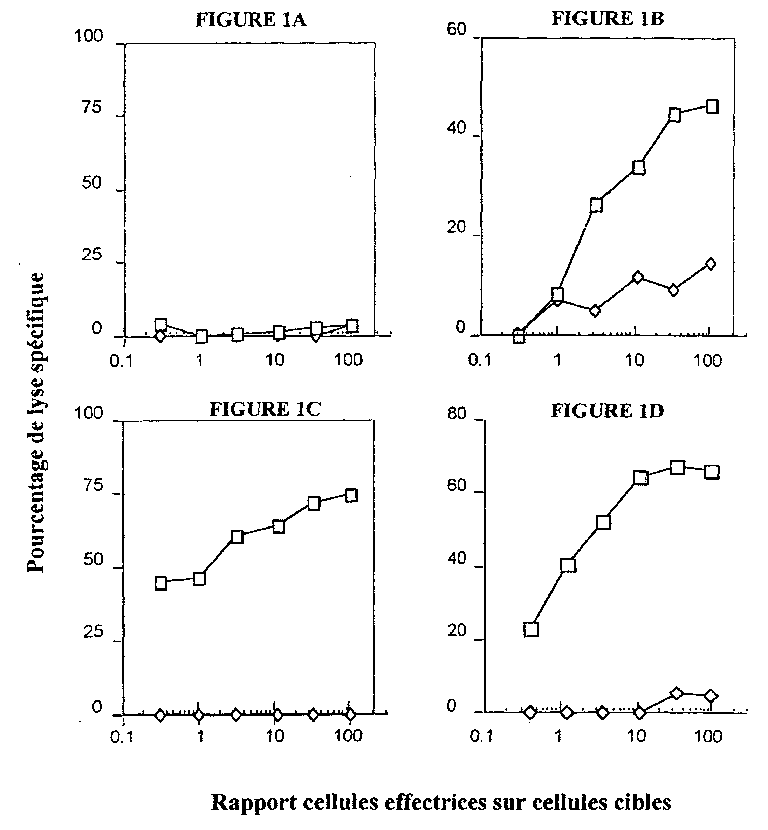

Figures 1A, 1B, 1C et 1D : Mesure de l'activité CTL anti-MELAN-A et anti-TRP-2 de cellules effectrices.

Après immunisation avec 50 μg hELA mélangé avec 3 μg rP40 (figure 1A), 50 μg hELA mélangé avec 300 μg rP40 (figure 1B), 50 μg hELA couplé à rP40 (figure 1C), ou 50 μg du peptide TRP-2 mélangé avec 300 μg rP40 (figure 1D), les cellules de ganglions drainants sont stimulées avec des cellules EL-4 A2/Kb (figures 1A, 1B, 1C) ou EL-4 (figure 1D) irradiées et pré-pulsées avec 1 μM du peptide relevant avant d'être évaluées pour leur capacité à tuer des cellules cibles pré-pulsées (rectangle) ou non (losange) avec le peptide relevant.

Les abscisses des repères des figures 1A à 1D correspondent au rapport des cellules T effectrices (lymphocytes activés) mises en présence sur les cellules cibles (EL-4 A2/Kb ou EL-4).

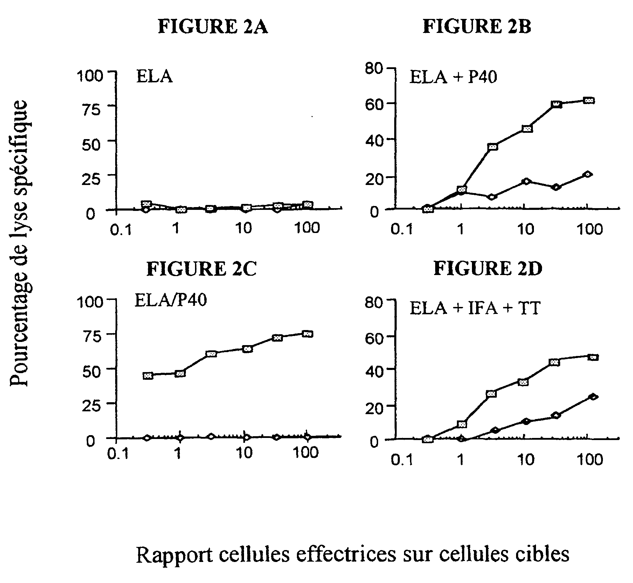

Figures 2A, 2B, 2C et 2D : Mesure de l'activité CTL anti-MELAN-A de cellules effectrices en présence de la protéine rP40 comparée à l'activité CTL obtenue avec un protocole standard d'immunisation.

Après immunisation avec HELA (50μg) seul (ELA, Figure 2A), hELA mélangé à 300 μg rP40 (ELA + P40, Figure 2B), hELA couplé à 300 μg rP40 (ELA/P40, Figure 2C) ou hELA mélangé avec 50μg de peptide P30 adjuvé avec IFA (ELA + IFA + TT, Figure 2D) (IFA pour Adjuvant Incomplet de Freund et TT pour Tetànus Toxoïd), les cellules de ganglions drainants sont stimulées deux semaines in vitro avec des cellules EL-4 A2/Kb irradiées et pré-pulsées avec 1 μM du peptide relevant avant d'être évaluées pour leur capacité à tuer des cellules cibles EL-4 A2/Kb prépulsées (rectangle) ou non (losange) avec le peptide hELA.

Exemple 1 : Clonage du gène codant pour la protéine P40 de *Klebsiella pneumoniae*

Le gène codant pour la protéine P40 a été obtenu par amplification par PCR à partir de l'ADN génomique de *Klebsiella pneumoniae* IP 1145 (Nguyen et col., Gene, 1998). Le fragment de gène codant de ce gène est inséré dans divers vecteurs d'expression sous contrôle de différents promoteurs, en particulier celui de l'opéron Trp. La séquence nucléotidique et la séquence peptidique de la protéine P40 sont représentées par les séquences SEQ ID N° 1 et SEQ ID N° 2 ci-après. Une souche productrice *E. coli* K12, a été transformée par un vecteur d'expression pvaLP40. La protéine recombinante P40 (dénommée rP40) est produite sous forme de corps d'inclusion avec un rendement important (> 10 % en g de protéines/g de biomasse sèche).

Cet exemple n'est qu'une illustration de l'expression de la protéine rP40, celle-ci pouvant être étendue à d'autres souches bactériennes ainsi qu'à d'autres vecteurs d'expression.

Exemple 2 : Procédé de fermentation de protéines de fusion rP40

Dans un erlenmeyer contenant 250 ml de milieu TSB (Tryptic Soy Broth, Difco) renfermant de l'Ampicilline (100 μg/ml, Sigma) et de la Tétracycline (8 μg/ml, Sigma) on inocule avec la souche *E. coli* transformée décrite ci-dessus. On incube pendant une nuit à 37°C puis, 200 ml de cette culture sont utilisés pour ensemencer 2 litres de milieu de culture dans un fermenteur (Biolafitte, France). De manière assez classique, le milieu de culture peut être composé d'agents chimiques, supplémentés par les vitamines, des extraits de levure, connus pour favoriser une croissance de cellules bactériennes à densité élevée.

Les paramètres contrôlés durant la fermentation sont : le pH, l'agitation, la température, le taux d'oxygénation, l'alimentation de sources combinées (glycérol ou glucose). De manière générale, le pH est régulé à 7,0, la température est fixée à 37°C. La croissance est contrôlée en alimentant en glycérol (87 %) à un débit constant (12 ml/h) pour maintenir le signal de tension de l'oxygène dissous à 30 %. Lorsque la turbidité de la culture (mesurée à 580 nm) atteint la valeur de 80 (après environ 24 heures de culture), la production des protéines est déclenchée par addition de l'acide indole acrylique (IAA) à la concentration finale de 25 mg/l. Environ 4 heures après induction, les cellules sont récoltées par centrifugation. La quantité de biomasse humide obtenue est d'environ 200 g.

Exemple 3 : Procédé d'extraction et de purification de la protéine rP40

Extraction de la rP40

Après centrifugation du bouillon de culture (4000 rpm, 10 min, 4°C), les cellules sont remises en suspension dans un tampon Tris-HCl, 25 mM, pH 8,5. Les insolubles, ou corps d'inclusion, sont obtenus après un traitement par le lysozyme (0,5 g/litre, 1 heure à température ambiante sous agitation douce). Le culot de corps d'inclusion obtenu par centrifugation (15 min à 10 000 g à 4°C) est repris dans un tampon Tris-HCl, 25 mM à pH 8,5 et 5 mM $MgCl_2$ puis centrifugé (15 min à 10 000 g).

On solubilise les corps d'inclusion à 37°C pendant 2 heures dans un tampon Tris-HCl, 25 mM, pH 8,5 contenant 7 M urée (agent dénaturant) et 10 mM de dithiothréitol (réduction des ponts disulfures). Une centrifugation (15 min à 10 000g) permet d'éliminer les particules non solubles.

On resuspend ensuite dans 13 X volumes de tampon Tris-HCl, 25 mM, pH 8,5 contenant du NaCl (8,76 g/l) et du Zwittergent 3-14 (0,1 %, p/v). La solution est laissée pendant une nuit à température ambiante sous agitation douce au contact de l'air (favoriser la renaturation de la protéine par dilution et réoxydation des ponts disulfures).

Purification de la protéine rP40

- Etape de chromatographie d'échange d'anions.

Après une nouvelle centrifugation, la solution est dialysée contre un tampon Tris-HCl, 25 mM, pH 8,5 contenant 0,1 % Zwittergent 3-14 (100 X volumes de tampon) pendant une nuit à 4°C.

Le dialysat est déposé sur une colonne contenant un support de type échangeur d'anions forts (gel Biorad Macro Prop High Q) équilibrée dans le tampon décrit ci-dessus, à un débit linéaire de 15 cm/h. Les protéines sont détectées à 280 nm. La protéine rP40 est éluée, avec un débit linéaire de 60 cm/h, pour une concentration de 0,2 M en NaCl dans le tampon Tris-HCl 25 mM, pH 8,5 : 0,1 % Zwittergent 3-14.

- Etape de chromatographie d'échange de cations.

Les fractions contenant la protéine rP40 sont poolées et concentrées par ultrafiltration à l'aide d'un système de cellule à agitation Amicon utilisé avec une membrane Diaflo de type YM10 (seuil de coupure 10 kDa) pour des volumes de l'ordre de 100 ml, ou à l'aide d'un système de filtration à flux tangentiel Minitan Millipore utilisé avec des plaques de membranes possédant un seuil de coupure 10 kDa pour des volumes supérieurs. La fraction ainsi concentrée est dialysée pendant une nuit à 4°C contre un tampon citrate 20 mM pH 3,0, à 0,1 % de Zwittergent 3-14.

Le dialysat est déposé sur une colonne contenant un support de type échangeur de cations forts (gel Biorad Macro Prep High S) équilibrée dans le tampon citrate 20 mM, pH 3,0, à 0,1 % de Zwittergent 3-14. La protéine rP40 est éluée (vitesse 61 cm/h) pour une concentration 0,7 M en NaCl. Les profils électrophorétiques montrent un degré de pureté de l'ordre de 95 %. L'état de la protéine est suivi par SDS-PAGE. Selon sa forme dénaturée ou native, la protéine P40, extraite de la membrane de *Klebsiella pneumoniae*, possède un comportement électrophorétique (migration) caractéristique. La forme native (structure en feuillets β) présente en effet une masse moléculaire plus faible que la forme dénaturée (structure en hélices α) sous l'action d'un agent dénaturant, tel que l'urée ou le chlorhydrate de guanidine, ou par chauffage à 100°C en présence de SDS. La protéine rP40 n'est pas correctement renaturée en fin de renaturation, que celle-ci soit réalisée en absence ou en présence de 0,1 % ; (p/v) Zwittergent 3-14. En revanche, une renaturation totale est obtenue après dialyse contre un tampon Tris/HCl 25 mM pH 8,5 contenant 0,1 % (p/v) Zwittergent 3-14. Toutefois, il faut noter que cette renaturation n'est obtenue que lorsque l'étape de dilution et le traitement à température ambiante sont réalisés eux-mêmes en présence de Zwittergent 3-14 (résultats négatifs en absence de détergent).

Exemple 4 : Génération de CTL

Les réponses CTL antitumorales dirigées contre les cellules de mélanome ont été définies pour plusieurs antigènes. Ces antigènes sont compris dans l'une de trois catégories :

a) les antigènes de rejet spécifiques du mélanome, tels que ceux de la famille des MAGE (revu par van der Bruggen et al., Science 254:1643) ;
b) les antigènes résultant de la mutation de protéines normales. Ce groupe inclut MUM-1 (Coulie et al., Proc. Natl.

Acad. Sci. USA 92:7976-7980 (1995) ; CDK4 (Wolfel et al., Science 296:1281-1284 (1995) et HLA-A2 (Brandel et al., J. Exp. Med. 183 :2501-2508 (1996) ;

c) les antigènes de différentiation, exprimés par les mélanomes et les mélanocytes. Ce groupe inclut la tyrosinase (Wolfel et al., Eur J. Immunol. 4:759 (1994) et Brichard et al., Eur. J. Immunol. 26:224 (1996)) ; gp 100 (Kang et al. , J. Immunol. 155:1343 (1995), Cox et al. , Science 264:716 (1994), et Kawakami et al. , J. Immunol. 155:3961 (1995)) ; gp75 (Wang et al., J. Exp. Med. 183:1131 (1996)), et Mart-1/MelanA (voir brevet US 5,620,886).

De tous ces antigènes, Mart-1/MelanA apparaît comme le meilleur candidat dans une utilisation en immunothérapie, et ce pour plusieurs raisons. D'abord, cet antigène a été identifié à partir de la réponse CTL in vivo des lymphocytes infiltrant le mélanome et non celle, in vitro, des cellules du sang périphérique, ce qui suggérerait une plus grande pertinence de cet antigène dans la réponse naturelle in vivo contre le mélanome (Kawakami et al., J. Exp. Med. 180:347 (1994)). De plus, Mart-1/MelanA est exprimé sur tous les mélanomes examinés, ce qui en fait une cible privilégiée d'une intervention immunothérapique. Enfin, des peptides dérivés de Mart-1/MelanA sont capables d'induire une réponse CTL spécifique chez les patients du mélanome exprimant l'antigène d'histocompatibilité HLA-A2 (Rivoltini et al., J. Immunol. 154:2257 (1995) ; Valmori et al. J. Immunol. 160:1750 (1998)).

HLA-A2 est l'allèle le plus fréquemment exprimé chez les Caucasiens. Les épitopes CTL de Mart-1/MelanA ont été définis pour cet allèle. Le peptide antigénique reconnu par la majorité des lignées CTL humaines comprend les acides aminés 27-35 AAGIGILTV (Kawakami et al., J. Exp. Med. 180:347 (1994)). D'autre part, des études sur l'affinité de la liaison avec HLA-A*0201 et la reconnaissance par des clones CTL ont démontré que le peptide optimal pour ces deux fonctions est le décapeptide 26-35 EAAGIGILTV (Romero et al., J. Immunol. 159:2366 (1997)). Cependant, il apparaît que ces peptides sont faiblement immunogéniques in vitro (Valmori et al., J. Immunol. 160:1750 (1998)) et in vivo (Jaeger et al., Int. J. Cancer 66:162 (1996)).

En comparant la séquence des acides aminés des épitopes T de Mart-1/MelanA avec les motifs peptidiques de A*0201 (Rammensee et al., Immunogenetics 41:178 (1995)), il apparaît que les peptides 26-35 et 27-35 ont des résidus d'ancrage non dominants à la position 2 et lient donc faiblement la molécule HLA-A*0201 (Kawakami et al., J. Immunol. 154:3961 (1995)), ce qui pourrait expliquer leur faible immunogénicité. La demande internationale de brevet publiée sous le numéro WO 98/58951 décrit un analogue au peptide 26-35 où l'Alanine en position 2 a été remplacée par une Leucine (séquence que l'on appellera ELA).

Le peptide hELA, utilisé dans les expériences ci-dessous, est l'objet de la demande de brevet WO 98/58951 propriété de l'Institut Ludwig de Recherche sur le Cancer. hELA est un analogue du décapeptide 26-35 (EAAGIGILTV) de Melan-A/MART-1, une protéine exprimée sur les mélanocytes et les mélanomes. Bien que le décapeptide 26-35 de Melan-AIMART-1 soit capable de se lier à la molécule HLA-A0201 (Romero et al., 1997, J. Immunol. 159, 2366-2374), il est faiblement immunogène in vitro et in vivo (Valmori et al., 1998, J. Immunol. 160, 1750-1758). L'analogue hELA a été généré par la substitution du second acide aminé du décapeptide 26-35 de Melan-A/MART-1 (une alanine) par une leucine. Le résultat de cette substitution, qui est basée sur une analyse des résidus nécessaire à l'ancrage des peptides à la molécule HLA-A0201, est une reconnaissance plus efficace par les CTL de patients de mélanome et une meilleure immunogénicité in vitro (Valmori et al., 1998, J. Immunol. 160, 1750-1758). Des souris transgéniques HLA-A* 0201/Kb (A2/Kb) de souche C57Bl/6 x BDA/2 (Vitiello et al., 1991, J. Exp. Med., 173, 1007-1015) ont été utilisées dans cette étude pour tester ELA. La molécule MHC de classe I exprimée chez ces souris est une molécule chimérique formée des domaines $\alpha$1 et a2 de la molécule humaine HLA-A0201 (allotype le plus fréquemment retrouvé) et du domaine $\alpha$3 de la molécule murine $K^b$.

Le peptide TRP-2 de séquence SEQ ID N° 4 est un octapeptide correspondant aux acides aminés 181-188 (VYDFFVWL) de la tyrosinase-related protein 2 (TRP-2). TRP-2 est exprimée dans les mélanocytes et les mélanomes. Il a été démontré que cet antigène induit des réponses CTL protectrices du mélanome dans les souris C57BL/6 (H-2$K^b$) (Bloom et al., 1997, J. Exp. Med. 185, 453-459).

A : Génération de CTL anti-Melan-A et anti-TRP-2 après immunisation par rP40 mélangé à un peptide analogue au Melan-A ou TRP-2

Protocole expérimental

Des souris A2/Kb ont reçu par injection sous-cutanée à la base de la queue :

-    50 μg ELA mélangés à 3 ou 300 μg rP40 ;
-    50 μg ELA couplés de façon covalente à 300 μg rP40.

Des souris C57BL/6 ont reçu par injection sous-cutanée à la base de la queue :

**-** 50 μg du peptide TRP-2 (181-188) mélangés à 300 μg rP40.

Génération de cellules cytotoxiques effectrices

10 jours après immunisation, les souris sont sacrifiées et les lymphocytes des ganglions drainant sont récupérés pour être stimulés in vitro avec le peptide relevant.
Ces lymphocytes (4 à 5 $10^6$) sont cultivés en plaque 24 puits en DMEM plus 10 mM HEPES, 10 % SVF et 50 μM β-2-mercaptoéthanol avec 2 à 5 $10^5$ cellules EL-4 A2/Kb ou EL4 irradiées (10 kRads) pré-pulsées 1 h à 37°C avec 1 μM du peptide relevant. Après deux stimulations hebdomadaires, les cellules sont testées pour leur activité cytotoxique.

Mesure de l'activité cytotoxique

Les cellules EL-4 A2/Kb ou EL4 sont incubées 1 h avec du $^{51}$Cr en présence ou non du peptide relevant, lavées puis co-incubées avec les cellules effectrices à différents ratio en plaque 96 puits dans un volume de 200 μl pendant 4 à 6h à 37°C. Les cellules sont ensuite centrifugées et le relargage de $^{51}$Cr est mesuré dans 100 μl de surnageant. Le pourcentage de lyse spécifique est calculé comme suit :

% lyse spécifique = (relargage expérimental-relargage spontané) / (relargage total -

relargage spontané) X 100.

Résultats

Comme le montrent les figures 1A à 1D, l'immunisation de souris avec une dose optimale de rP40 (300 μg) en mélange avec hELA (figure 1B) ou TRP-2 (figure 1D) induit une forte réponse CTL spécifique. Une telle réponse est également observée après immunisation avec rP40 couplée à hELA (figure 1C). Par contre, l'immunisation avec le peptide seul ou rP40 seule (résultats non montrés) ou avec le peptide hELA en mélange avec une dose sous-optimale de rP40 (3 μg) n'induit aucune activité CTL (figure 1A). Ces résultats démontrent que la molécule rP40 mélangée ou couplée à des peptides immunogènes permet d'induire une réponse CTL spécifique in vivo, et ce sans l'ajout d'adjuvant.

B : Génération de CTL anti-Melan-A après immunisation par rP40 mélangé à un peptide analogue au Melan-A comparée à un protocole standard d'immunisation

Protocole expérimental

Des souris A2/Kb ont reçu :

**-** 50μl d'IFA (adjuvant incomplet de Freud) en injection sous-cutanée à la base de la queue puis 3 semaines après 50μg de hELA en présence de 50μg d'un peptide p30 T helper dérivé de Tetanus Toxoïd (TT) (Panina-Bordignon et al., Eur. J. Immunol., 1989, 19, 2237) adjuvé en IFA. Ce protocole a été décrit pour permettre de générer des CTL anti-peptides (Valmori et al., Eur. J. Immunol., 1994, 24, 1458) et sert de contrôle positif.
**-** 50μg de hELA seul ou 300μg de rP40 mélangé ou couplé à 50 μg hELA.

Génération de cellules cytotoxiques effectrices

10 jours après la dernière immunisation, les souris sont sacrifiées et les lymphocytes des ganglions drainant sont récupérés pour être stimulés in vitro avec le peptide relevant.
Ces lymphocytes (4 à 5 $10^6$) sont cultivés en plaque 24 puits en DMEM plus 10 mM HEPES, 10% SVF et 50 μM β-2-mercaptoethanol avec 2 à 5 $10^5$ cellules EL-4 A2/Kb (cellules murines transfectées avec le gène HLA-A* 0201/Kb) irradiées (10 kRads) pré-pulsées 1 h à 37°C avec 1μM du peptide relevant.
Après une, deux ou trois stimulations hebdomadaires, les cellules sont testées pour leur activité cytotoxique.
La mesure de l'activité cytotoxique est effectuée selon la méthode décrite précédemment. Résultats
Une activité CTL anti-hELA est mesurée après immunisation par rP40 couplée à hELA non adjuvé comparable à celle observée après immunisation avec hELA + P30/IFA (cf. figures 2C et 2D). De même, le mélange rP40 + peptide hELA lui aussi non adjuvé génère des CTL de manière similaire à ce qui est obtenu avec un protocole classique de génération de CTL (cf. figures 2B et 2D).

Aucune activité CTL n'a été détectée après immunisation avec le peptide seul (cf. figure 2A), ou la protéine rP40 seule (non montrée) indépendamment du jour de stimulation des cellules effectrices.

**Revendications**

**1.** Utilisation d'une protéine OmpA d'entérobactérie associée au peptide de séquence SEQ ID N° 3 ELAGIGILTV, pour la préparation d'une composition pharmaceutique destinée au traitement ou à la prévention des mélanomes malins.

**2.** Utilisation selon la revendication 1, **caractérisée en ce que** ladite protéine OmpA d'entérobactérie est obtenue par un procédé d'extraction à partir d'une culture de ladite entérobactérie.

**3.** Utilisation selon la revendication 1, **caractérisée en ce que** ladite protéine OmpA d'entérobactérie est obtenue par voie recombinante.

**4.** Utilisation selon l'une des revendications 1 à 3, **caractérisée en ce que** ladite entérobactérie est *Klebsiella pneumoniae.*

**5.** Utilisation selon la revendication 4, **caractérisée en ce que** la séquence d'acides aminés de ladite protéine OmpA comprend :

a) la séquence d'acides aminés de séquence SEQ ID N° 2 ; ou
b) la séquence d'acides aminés d'une séquence présentant une homologie d'au moins 80 % avec la séquence SEQ ID N° 2.

**6.** Utilisation selon l'une des revendications 1 à 5, **caractérisée en ce que** ledit peptide de séquence SEQ ID N° 3 est couplé ou mélangé avec ladite protéine OmpA.

**7.** Utilisation selon la revendication 5, **caractérisée en ce que** ledit peptide de séquence SEQ ID N° 3 est couplé par liaison covalente avec ladite protéine OmpA.

**8.** Utilisation selon la revendication 7, **caractérisée en ce que** le couplage par liaison covalente est un couplage réalisé par synthèse chimique.

**9.** Utilisation selon la revendication 8, **caractérisée en ce qu'**il est introduit un ou plusieurs éléments de liaison dans ladite protéine OmpA et/ou dans ledit peptide de séquence SEQ ID N° 3 pour faciliter le couplage chimique.

**10.** Utilisation selon la revendication 9, **caractérisée en ce que** ledit élément de liaison introduit est un acide aminé.

**11.** Utilisation selon la revendication 7, **caractérisée en ce que** la protéine hybride résultant du couplage entre ledit peptide de séquence SEQ ID N° 3 et ladite protéine OmpA est obtenue par recombinaison génétique.

**12.** Utilisation selon la revendication 11, **caractérisée en ce que** la composition pharmaceutique comprend une séquence nucléique codant pour ladite protéine hybride.

**13.** Utilisation selon la revendication 12, **caractérisée en ce que** ladite sequence nucléique est contenue dans un vecteur, ou dans une cellule hôte transformée capable d'exprimer ladite protéine hybride.

**14.** Utilisation selon l'une des revendications 1 à 13, pour la préparation d'une composition pharmaceutique administrable par voie sous-cutanée ou intradermique.

**15.** Utilisation selon l'une des revendications 1 à 14, **caractérisée en ce que** ladite composition pharmaceutique est véhiculée sous une forme permettant d'améliorer sa stabilité et/ou son immunogénicité.

**16.** Composition pharmaceutique, **caractérisée en ce qu'**elle comprend la protéine OmpA de *Klebsiella pneumoniae* de séquence SEQ ID N° 2 ou une protéine dont la séquence présente une homologie d'au moins 80 % avec la séquence SEQ ID N° 2, associée par mélange ou par couplage au peptide de séquence SEQ ID N° 3.

**17.** Composition pharmaceutique **caractérisée en ce qu'**elle comprend une séquence nucléique contenant un acide nucléique codant pour la protéine OmpA de *Klebsiella pneumoniae* de séquence SEQ ID N° 2 ou pour une protéine dont la séquence présente une homologie d'au moins 80 % avec la séquence SEQ ID N° 2, et un acide nucléique codant pour le peptide de séquence SEQ ID N° 3.

**18.** Composition selon l'une des revendications 16 et 17, **caractérisée en ce que** ladite composition pharmaceutique est véhiculée sous une forme permettant d'améliorer sa stabilité et/ou son immunogénicité.

**19.** Composition selon la revendication 18, **caractérisée en ce que** ledit véhicule est un liposome, ou un vecteur viral ou une cellule hôte transformée capable d'exprimer ladite protéine OmpA, et ledit peptide de séquence SEQ ID N° 3.

**20.** Composition selon l'une des revendications 16 à 19, **caractérisée en ce que** ladite composition est contenue dans un milieu pharmaceutiquement acceptable.

**21.** Composition selon l'une des revendications 16 à 20, **caractérisée en ce que** ladite composition contient en outre un détergent.

**22.** Composition selon l'une des revendications 16 à 22, sans autre adjuvant permettant d'induire une réponse CTL.

**Patentansprüche**

1. Verwendung eines Enterobakterium-OmpA-Proteins, das mit dem Peptid der Sequenz SEQ ID NO: 3 ELAGIGILTV assoziiert ist, für die Herstellung einer pharmazeutischen Zusammensetzung, die zur Behandlung oder Verhütung von malignen Melanomen bestimmt ist.

2. Verwendung nach Anspruch 1, **dadurch gekennzeichnet, dass** das Enterobakterium-OmpA-Protein durch ein Extraktionsverfahren aus einer Kultur des Enterobakteriums erhalten wird.

3. Verwendung nach Anspruch 1, **dadurch gekennzeichnet, dass** das Enterobakterum-OmpA-Protein auf rekombinantem Weg erhalten wird.

4. Verwendung nach einem der Ansprüche 1 bis 3, **dadurch gekennzeichnet, dass** das Enterobakterium *Klebsiella pneumoniae* ist.

5. Verwendung nach Anspruch 4, **dadurch gekennzeichnet, dass** die Aminosäuresequenz des OmpA-Proteins umfasst:

   a) die Aminosäuresequenz der Sequenz SEQ ID NO: 2; oder

   b) die Aminosäuresequenz einer Sequenz, die eine Homologie von mindestens 80 % zu der Sequenz SEQ ID NO: 2 aufweist.

6. Verwendung nach einem der Ansprüche 1 bis 5, **dadurch gekennzeichnet, dass** das Peptid der Sequenz SEQ ID NO: 3 mit dem OmpA-Protein gekuppelt oder gemischt ist.

7. Verwendung nach Anspruch 5, **dadurch gekennzeichnet, dass** das Peptid der Sequenz SEQ ID NO: 3 durch kovalente Bindung mit dem OmpA-Protein gekuppelt ist.

8. Verwendung nach Anspruch 7, **dadurch gekennzeichnet, dass** die Kupplung durch kovalente Bindung eine Kupplung ist, die durch chemische Synthese bewirkt wird.

9. Verwendung nach Anspruch 8, **dadurch gekennzeichnet, dass** ein oder mehrere Bindungselemente in das OmpA-Protein und/oder in das Peptid der Sequenz SEQ ID NO: 3 eingeführt sind, um die chemische Kupplung zu erleichtern.

10. Verwendung nach Anspruch 9, **dadurch gekennzeichnet, dass** das Bindungselement eine Aminosäure ist.

11. Verwendung nach Anspruch 7, **dadurch gekennzeichnet, dass** das Hybridprotein, das aus der Kupplung zwischen dem Peptid der Sequenz SEQ ID NO: 3 und dem OmpA-Protein resultiert, durch genetische Rekombination erhalten wird.

12. Verwendung nach Anspruch 11, **dadurch gekennzeichnet, dass** die pharmazeutische Zusammensetzung eine Nukleotidsequenz enthält, die das Hybridprotein codiert.

13. Verwendung nach Anspruch 12, **dadurch gekennzeichnet, dass** die Nukleotidsequenz in einem Vektor oder in einer transformierten Wirtszeiie enthalten ist, die das Hybridprotein exprimieren kann.

14. Verwendung nach einem der Ansprüche 1 bis 13 für die Herstellung einer pharmazeutischen Zusammensetzung, die auf subkutanem oder intradermalem Weg verabreichbar ist.

15. Verwendung nach einem der Ansprüche 1 bis 14, **dadurch gekennzeichnet, dass** die pharmazeutische Zusammensetzung mit einem Vehikel in einer Form versehen ist, welche gestattet, deren Stabilität und/oder deren Immunogenität zu verbessern.

16. Pharmazeutische Zusammensetzung, **dadurch gekennzeichnet, dass** sie das OmpA-Protein von *Klebsiella pneumoniae* der Sequenz SEQ ID NO: 2 oder ein Protein umfasst, dessen Sequenz eine Homologie von mindestens 80 % zu der Sequenz SEQ ID NO: 2 aufweist, welches durch Mischen oder durch Kuppeln mit dem Peptid

17

der Sequenz SEQ ID NO: 3 assoziiert ist.

**17.** Pharmazeutische Zusammensetzung, **dadurch gekennzeichnet, dass** sie eine Nukleotidsequenz umfasst, die eine Nukleinsäure, welche das OmpA-Protein von *Klebsiella pneumoniae* der Sequenz SEQ ID NO: 2 oder ein Protein codiert, dessen Sequenz eine Homologie von mindestens 80 % zu der Sequenz SEQ ID NO: 2 aufweist, und eine Nukleinsäure enthält, welche das Peptid der Sequenz SEQ ID NO: 3 codiert.

**18.** Zusammensetzung nach einem der Ansprüche 16 und 17, **dadurch gekennzeichnet, dass** die pharmazeutische Zusammensetzung mit einem Vehikel in einer Form versehen ist, welche gestattet, deren Stabilität und/oder deren Immunogenität zu verbessern.

**19.** Zusammensetzung nach Anspruch 18, **dadurch gekennzeichnet, dass** das Vehikel ein Liposom oder ein viraler Vektor oder eine transformierte Wirtszelle ist, die das OmpA-Protein und das Peptid der Sequenz SEQ ID NO: 3 exprimieren kann.

**20.** Zusammensetzung nach einem der Ansprüche 16 bis 19, **dadurch gekennzeichnet, dass** die Zusammensetzung in einem pharmazeutisch annehmbaren Medium enthalten ist.

**21.** Zusammensetzung nach einem der Ansprüche 16 bis 20, **dadurch gekennzeichnet, dass** die Zusammensetzung weiter ein Detergens enthält.

**22.** Zusammensetzung nach einem der Ansprüche 16 bis 22 ohne weiteres Adjuvans, welches ermöglicht, eine CTL-Reaktion zu induzieren.

**Claims**

**1.** Use of an enterobacterium OmpA protein, combined with the peptide of sequence SEQ ID No. 3 ELAGIGILTV, for preparing a pharmaceutical composition intended for treating or preventing malignant melanomas.

**2.** Use according to Claim 1, **characterized in that** said enterobacterium OmpA protein is obtained using a method of extraction from a culture of said enterobacterium.

**3.** Use according to Claim 1, **characterized in that** said enterobacterium OmpA protein is obtained via the recombinant route.

**4.** Use according to one of Claims 1 to 3, **characterized in that** said enterobacterium is *Klebsiella pneumoniae*.

**5.** Use according to Claim 4, **characterized in that** the amino acid sequence of said OmpA protein comprises:

   a) the amino acid sequence of sequence SEQ ID No. 2; or
   b) the amino acid sequence of a sequence having at least 80% homology with the sequence SEQ ID No. 2.

**6.** Use according to one of Claims 1 to 5, **characterized in that** said peptide of sequence SEQ ID No. 3 is coupled to or mixed with said OmpA protein.

**7.** Use according to Claim 5, **characterized in that** said peptide of sequence SEQ ID No. 3 is coupled, by covalent attachment, with said OmpA protein.

**8.** Use according to Claim 7, **characterized in that** the coupling by covalent attachment is coupling produced by chemical synthesis.

**9.** Use according to Claim 8, **characterized in that** one or more attachment elements is(are) introduced into said OmpA protein and/or into said peptide of sequence SEQ ID No. 3, in order to facilitate the chemical coupling.

**10.** Use according to Claim 9, **characterized in that** said attachment element introduced is an amino acid.

**11.** Use according to Claim 7, **characterized in that** the hybrid protein resulting from the coupling between said peptide of sequence SEQ ID No. 3 and said OmpA protein is obtained by genetic recombination.

12. Use according to Claim 11, **characterized in that** the pharmaceutical composition comprises a nucleic acid sequence encoding said hybrid protein.

13. Use according to Claim 12, **characterized in that** said nucleic acid sequence is contained in a vector, or in a transformed host cell capable of expressing said hybrid protein.

14. Use according to one of Claims 1 to 13, for preparing a pharmaceutical composition which can be administered by the subcutaneous or intradermal route.

15. Use according to one of Claims 1 to 14, **characterized in that** said pharmaceutical composition is vehicled in a form which makes it possible to improve its stability and/or its immunogenicity.

16. Pharmaceutical composition, **characterized in that** it comprises the *Klebsiella pneumoniae* OmpA protein of sequence SEQ ID No. 2 or a protein, the sequence of which has at least 80% homology with the sequence SEQ ID No. 2, combined, by mixing or by coupling, with the peptide of sequence SEQ ID No. 3.

17. Pharmaceutical composition, **characterized in that** it comprises a nucleic acid sequence containing a nucleic acid encoding the *Klebsiella pneumoniae* OmpA protein of sequence SEQ ID No. 2 or encoding a protein, the sequence of which has at least 80% homology with sequence SEQ ID No. 2, and a nucleic acid encoding the peptide of sequence SEQ ID No. 3.

18. Composition according to one of Claims 16 and 17, **characterized in that** said pharmaceutical composition is vehicled in a form which makes it possible to improve its stability and/or its immunogenicity.

19. Composition according to Claim 18, **characterized in that** said vehicle is a liposome, or a viral vector or a transformed host cell capable of expressing said OmpA protein and said peptide of sequence SEQ ID No. 3.

20. Composition according to one of Claims 16 to 19, **characterized in that** said composition is contained in a pharmaceutically acceptable medium.

21. Composition according to one of Claims 16 to 20, **characterized in that** said composition also contains a detergent.

22. Composition according to one of Claims 16 to 21, without any other adjuvant for inducing a CTL response.

**FIGURE 1A**

**FIGURE 1B**

**FIGURE 1C**

**FIGURE 1D**

**Pourcentage de lyse spécifique**

**Rapport cellules effectrices sur cellules cibles**

## FIGURE 2A

## FIGURE 2B

## FIGURE 2C

## FIGURE 2D

Rapport cellules effectrices sur cellules cibles


LISTE DE SEQUENCES

<110> PIERRE FABRE MEDICAMENT

<120> UTILISATION D'UNE PROTEINE OmpA D' ENTEROBACTERIE ASSOCIEE AU PEPTIDE ELAGIGILTV POUR LE TRAITEMENT DES MELANOMES.

<130> D18441

<150> FR 99 01917
<151> 1999-02-17

<160> 4

<170> PatentIn Ver. 2.1

<210> 1
<211> 1035
<212> ADN
<213> Klebsiella pneumoniae

<220>
<221> exon
<222> (1)..(1032)

<220>
<221> intron
<222> (1033)..(1035)

<220>
<221> CDS
<222> (1)..(1032)

<400> 1

```
atg aaa gca att ttc gta ctg aat gcg gct ccg aaa gat aac acc tgg    48
Met Lys Ala Ile Phe Val Leu Asn Ala Ala Pro Lys Asp Asn Thr Trp
 1               5                  10                  15

tat gca ggt ggt aaa ctg ggt tgg tcc cag tat cac gac acc ggt ttc    96
Tyr Ala Gly Gly Lys Leu Gly Trp Ser Gln Tyr His Asp Thr Gly Phe
                20                  25                  30

tac ggt aac ggt ttc cag aac aac aac ggt ccg acc cgt aac gat cag   144
Tyr Gly Asn Gly Phe Gln Asn Asn Asn Gly Pro Thr Arg Asn Asp Gln
             35                  40                  45

ctt ggt gct ggt gcg ttc ggt ggt tac cag gtt aac ccg tac ctc ggt   192
Leu Gly Ala Gly Ala Phe Gly Gly Tyr Gln Val Asn Pro Tyr Leu Gly
         50                  55                  60

ttc gaa atg ggt tat gac tgg ctg ggc cgt atg gca tat aaa ggc agc   240
Phe Glu Met Gly Tyr Asp Trp Leu Gly Arg Met Ala Tyr Lys Gly Ser
 65                  70                  75                  80

gtt gac aac ggt gct ttc aaa gct cag ggc gtt cag ctg acc gct aaa   288
Val Asp Asn Gly Ala Phe Lys Ala Gln Gly Val Gln Leu Thr Ala Lys
                    85                  90                  95

ctg ggt tac ccg atc act gac gat ctg gac atc tac acc cgt ctg ggc   336
Leu Gly Tyr Pro Ile Thr Asp Asp Leu Asp Ile Tyr Thr Arg Leu Gly
```

            100                      105                  110

```
ggc atg gtt tgg cgc gct gac tcc aaa ggc aac tac gct tct acc ggc    384
Gly Met Val Trp Arg Ala Asp Ser Lys Gly Asn Tyr Ala Ser Thr Gly
        115              120              125

gtt tcc cgt agc gaa cac gac act ggc gtt tcc cca gta ttt gct ggc    432
Val Ser Arg Ser Glu His Asp Thr Gly Val Ser Pro Val Phe Ala Gly
    130              135              140

ggc gta gag tgg gct gtt act cgt gac atc gct acc cgt ctg gaa tac    480
Gly Val Glu Trp Ala Val Thr Arg Asp Ile Ala Thr Arg Leu Glu Tyr
145              150              155              160

cag tgg gtt aac aac atc ggc gac gcg ggc act gtg ggt acc cgt cct    528
Gln Trp Val Asn Asn Ile Gly Asp Ala Gly Thr Val Gly Thr Arg Pro
                 165              170              175

gat aac ggc atg ctg agc ctg ggc gtt tcc tac cgc ttc ggt cag gaa    576
Asp Asn Gly Met Leu Ser Leu Gly Val Ser Tyr Arg Phe Gly Gln Glu
             180              185              190

gat gct gca ccg gtt gtt gct ccg gct ccg gct ccg gct ccg gaa gtg    624
Asp Ala Ala Pro Val Val Ala Pro Ala Pro Ala Pro Ala Pro Glu Val
         195              200              205

gct acc aag cac ttc acc ctg aag tct gac gtt ctg ttc aac ttc aac    672
Ala Thr Lys His Phe Thr Leu Lys Ser Asp Val Leu Phe Asn Phe Asn
    210              215              220

aaa gct acc ctg aaa ccg gaa ggt cag cag gct ctg gat cag ctg tac    720
Lys Ala Thr Leu Lys Pro Glu Gly Gln Gln Ala Leu Asp Gln Leu Tyr
225              230              235              240

act cag ctg agc aac atg gat ccg aaa gac ggt tcc gct gtt gtt ctg    768
Thr Gln Leu Ser Asn Met Asp Pro Lys Asp Gly Ser Ala Val Val Leu
                 245              250              255

ggc tac acc gac cgc atc ggt tcc gaa gct tac aac cag cag ctg tct    816
Gly Tyr Thr Asp Arg Ile Gly Ser Glu Ala Tyr Asn Gln Gln Leu Ser
             260              265              270

gag aaa cgt gct cag tcc gtt gtt gac tac ctg gtt gct aaa ggc atc    864
Glu Lys Arg Ala Gln Ser Val Val Asp Tyr Leu Val Ala Lys Gly Ile
         275              280              285

ccg gct ggc aaa atc tcc gct cgc ggc atg ggt gaa tcc aac ccg gtt    912
Pro Ala Gly Lys Ile Ser Ala Arg Gly Met Gly Glu Ser Asn Pro Val
     290              295              300

act ggc aac acc tgt gac aac gtg aaa gct cgc gct gcc ctg atc gat    960
Thr Gly Asn Thr Cys Asp Asn Val Lys Ala Arg Ala Ala Leu Ile Asp
305              310              315              320

tgc ctg gct ccg gat cgt cgt gta gag atc gaa gtt aaa ggc tac aaa   1008
Cys Leu Ala Pro Asp Arg Arg Val Glu Ile Glu Val Lys Gly Tyr Lys
                 325              330              335

gaa gtt gta act cag ccg gcg ggt taa                               1035
Glu Val Val Thr Gln Pro Ala Gly
                 340
```

<210> 2
<211> 344
<212> PRT
<213> Klebsiella pneumoniae

<400> 2

```
Met Lys Ala Ile Phe Val Leu Asn Ala Ala Pro Lys Asp Asn Thr Trp
 1               5                  10                  15

Tyr Ala Gly Gly Lys Leu Gly Trp Ser Gln Tyr His Asp Thr Gly Phe
             20                  25                  30

Tyr Gly Asn Gly Phe Gln Asn Asn Asn Gly Pro Thr Arg Asn Asp Gln
             35                  40                  45

Leu Gly Ala Gly Ala Phe Gly Gly Tyr Gln Val Asn Pro Tyr Leu Gly
         50                  55                  60

Phe Glu Met Gly Tyr Asp Trp Leu Gly Arg Met Ala Tyr Lys Gly Ser
 65                  70                  75                  80

Val Asp Asn Gly Ala Phe Lys Ala Gln Gly Val Gln Leu Thr Ala Lys
                 85                  90                  95

Leu Gly Tyr Pro Ile Thr Asp Asp Leu Asp Ile Tyr Thr Arg Leu Gly
             100                 105                 110

Gly Met Val Trp Arg Ala Asp Ser Lys Gly Asn Tyr Ala Ser Thr Gly
         115                 120                 125

Val Ser Arg Ser Glu His Asp Thr Gly Val Ser Pro Val Phe Ala Gly
     130                 135                 140

Gly Val Glu Trp Ala Val Thr Arg Asp Ile Ala Thr Arg Leu Glu Tyr
145                 150                 155                 160

Gln Trp Val Asn Asn Ile Gly Asp Ala Gly Thr Val Gly Thr Arg Pro
                 165                 170                 175

Asp Asn Gly Met Leu Ser Leu Gly Val Ser Tyr Arg Phe Gly Gln Glu
             180                 185                 190

Asp Ala Ala Pro Val Val Ala Pro Ala Pro Ala Pro Ala Pro Glu Val
         195                 200                 205

Ala Thr Lys His Phe Thr Leu Lys Ser Asp Val Leu Phe Asn Phe Asn
     210                 215                 220

Lys Ala Thr Leu Lys Pro Glu Gly Gln Gln Ala Leu Asp Gln Leu Tyr
225                 230                 235                 240

Thr Gln Leu Ser Asn Met Asp Pro Lys Asp Gly Ser Ala Val Val Leu
             245                 250                 255

Gly Tyr Thr Asp Arg Ile Gly Ser Glu Ala Tyr Asn Gln Gln Leu Ser
         260                 265                 270

Glu Lys Arg Ala Gln Ser Val Val Asp Tyr Leu Val Ala Lys Gly Ile
     275                 280                 285
```

```
Pro Ala Gly Lys Ile Ser Ala Arg Gly Met Gly Glu Ser Asn Pro Val
    290                 295             300

Thr Gly Asn Thr Cys Asp Asn Val Lys Ala Arg Ala Ala Leu Ile Asp
305                 310                 315                 320

Cys Leu Ala Pro Asp Arg Arg Val Glu Ile Glu Val Lys Gly Tyr Lys
                325                 330                 335

Glu Val Val Thr Gln Pro Ala Gly
                340
```

<210> 3
<211> 10
<212> PRT
<213> Homo sapiens


<220>
<223> Peptide dérivé de l'antigène Mart-1/MelanA exprimé par les cellules de mélanome.


<400> 3

```
Glu Leu Ala Gly Ile Gly Ile Leu Thr Val
 1               5               10
```


<210> 4
<211> 8
<212> PRT
<213> Homo sapiens


<220>
<223> Dérivé de 1a tyrosinase-related protein 2 (TRP-2).


<400> 4

```
Val Tyr Asp Phe Phe Val Trp Leu
 1               5
```